(19) Europäisches Patentamt
European Patent Office
Office européen des brevets

(11) Publication number: **0 008 188**
**B1**

(12) **EUROPEAN PATENT SPECIFICATION**

(45) Date of publication of patent specification: **23.06.82**

(51) Int. Cl.[3]: **C 09 K 3/34,** C 07 C 121/52

(21) Application number: **79301523.1**

(22) Date of filing: **31.07.79**

(54) **Liquid crystal compounds and materials and devices containing them.**

(30) Priority: **04.08.78 GB 3235078**

(43) Date of publication of application:
**20.02.80 Bulletin 80/4**

(45) Publication of the grant of the patent:
**23.06.82 Bulletin 82/25**

(84) Designated Contracting States:
**CH DE FR IT NL**

(56) References cited:
**none**

(73) Proprietor: **The Secretary of State for Defence in Her Britannic Majesty's Government of The United Kingdom of Great Britain and Northern Ireland Whitehall London SW1A 2HB (GB)**

(72) Inventor: **Gray, George William**
**49 Kingtree Avenue**
**Cottingham, Humberside (GB)**
Inventor: **Kelly, Stephen Malcolm**
**17 Hamlyn Avenue**
**Anlaby Road, Hull, Humberside (GB)**
Inventor: **McDonnell, Damien Gerard**
**34 Britten Drive**
**Poolbrook, Malvern Worcester (GB)**
Inventor: **Mosley, Alan**
**149 Colemans Moor Road**
**Woodley, Reading, Berkshire (GB)**

(74) Representative: **McCormack, Derek James et al,**
**Procurement Executive Ministry of Defence**
**Patents 1A(4) Room 1932 Empress State Building Lillie Road**
**London SW6 1TR (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 008 188 B1

Liquid crystal compounds and materials and devices containing them

The present invention relates to liquid crystal compounds and materials and devices containing them.

In the field of displays there is a requirement for electro-optic devices having a low power consumption. Devices incorporating liquid crystal materials have been shown to satisfy this requirement because they have an electrically switchable molecular arrangement and a very large electrical resistance, and at the present time a considerable amount of interest is being shown in such devices for those reasons.

There are many known liquid crystal materials; some have been known for many years. Liquid crystal materials are organic materials which exhibit a liquid crystal phase in which the molecules are arranged over limited spatial ranges in an ordered structure.

The materials may be single compounds or mixtures of compounds.

According to the present invention there is provided a liquid crystal compound having the following molecular structure:

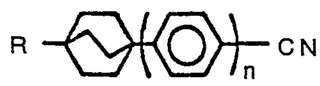 (I)

where R is an alkyl group.

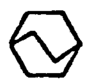

is a bicyclo (2,2,2) octane ring,

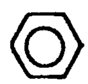

is a phenyl ring, CN is a cyano group and n is an integer greater than 0.

A compound (or compounds) having molecular structure (I) will be referred to as a compound (or compounds), as defined.

The alkyl group R preferably contains less than eighteen carbon atoms, e.g. one to ten carbon atoms, and may be normal or branched. If R is branched it may contain a chiral centre in which case the compound is optically active.

Preferably n is 1 or 2 so that the compound is either

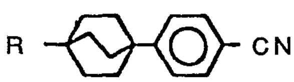 or 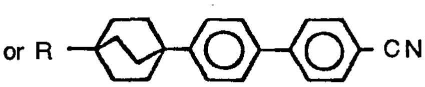

By a 'liquid crystal compound' is meant a compound in one of the following two known categories:

(i) Compounds which normally exhibit a liquid crystal phase;

(ii) Compounds which do not normally exhibit a liquid crystal phase but which nevertheless usefully affect some aspect of liquid crystal behaviour when dissolved in other liquid crystal compounds.

Compounds in category (ii) show a 'monotropic' or 'virtual' liquid crystal to isotropic liquid transition at a temperature below the melting point of their solid phase. The monotropic or virtual transition may be detected respectively by rapid cooling of the liquid phase or by dissolving the compound in a material exhibiting a liquid crystal phase, observing the change in the transition to the isotropic liquid phase of the material by the addition and calculating the virtual transition temperature by extrapolation.

Compounds in category (ii) might for example be usefully dissolved in other liquid crystal compounds to extend or vary the liquid crystal temperature ranges of the compounds or to vary the molecular helical pitch (in the case of 'cholesteric' liquid crystals).

Material which exhibits a liquid crystal phase and either consists of or contains a compound as defined above will be referred to herein as a material 'as defined'.

The liquid crystal phase exhibited by the material as defined may be nematic, smectic or cholesteric.

For a nematic phase the compound as defined should have a normal alkyl group R whilst for a cholesteric phase the compound as defined should have a branched alkyl group R containing a chiral centre.

Preferably compounds as defined are prepared from the intermediate bromo compound

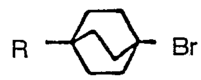

e.g. by one of the following routes

(i)

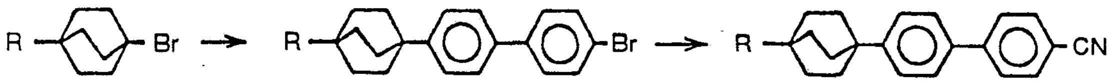

(ii)

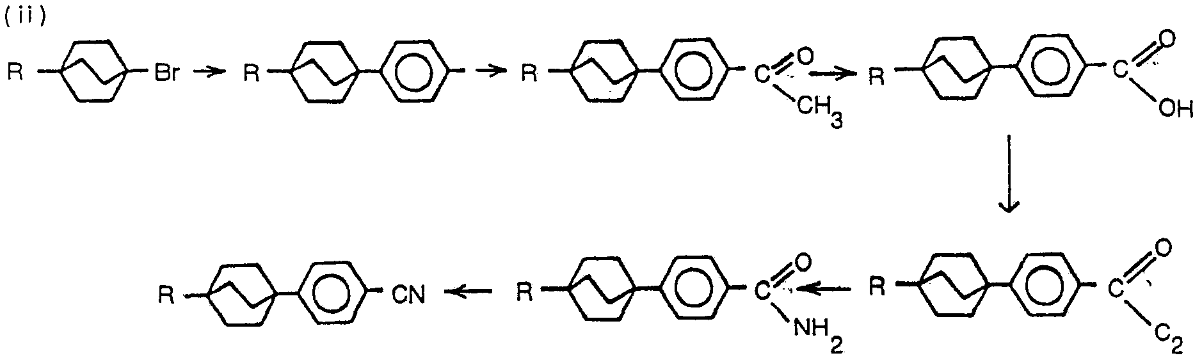

A compound as defined may be mixed with one or more other compounds as defined to enhance its liquid crystal properties, e.g. to extend its liquid crystal temperature range.

For example the following are examples of such mixtures:

(i) 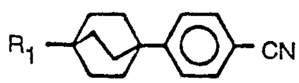 plus 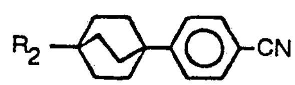

(ii) 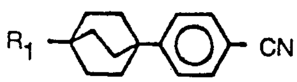 plus 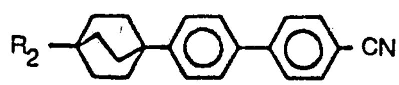

(iii) 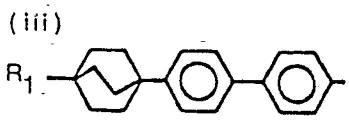 plus 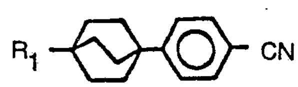 plus 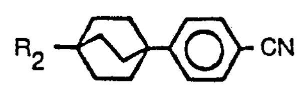

$R_1$, $R_2$, $R_3$ = alkyl $R_1 \neq R_2$

Likewise, compounds as defined may be mixed with one or more of the compounds in the following categories:

(i) 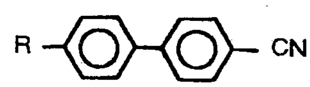

(ii) 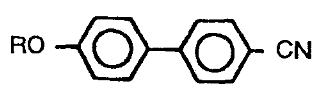

(iii) 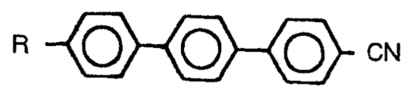

(iv) 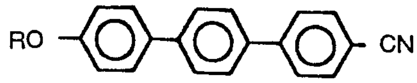

(v) 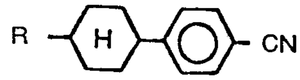

(vi) 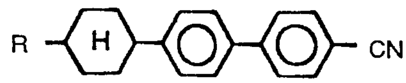

(vii) 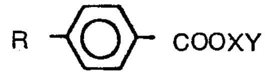

(viii) 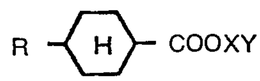

(ix) 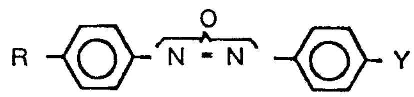

(x) 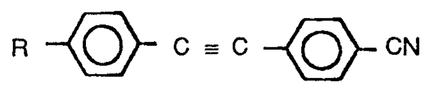

(xi) 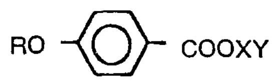

(xii) 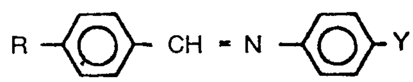

(xiii) 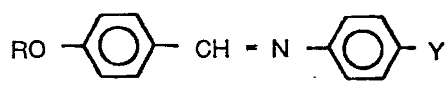

(xiv) 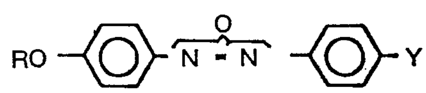

(xv) 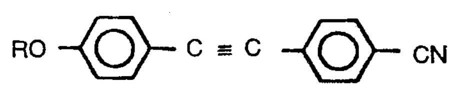

where

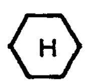

is a cyclohexane ring, X is a 1,4 phenylene group

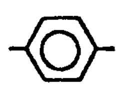

or a 4,4′ biphenyl group

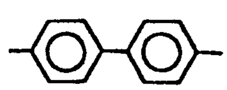

or a 2,6 naphthyl group

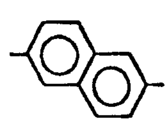

and Y is CN, or $R^1$, or $OR^1$ or CO.O—X—$Y^1$ where $Y^1$ is CN, or $R'$ or $OR'$; the definition of $R'$ being the same as that of R.

According to another aspect of the present invention a liquid crystal device includes means for containing a region of liquid crystal material, a region of liquid crystal material contained in the containing means, wherein the liquid crystal material is material as defined above, and means for applying an external stimulus to the material to alter the molecular arrangement in the material.

The means for applying an external stimulus may for example be means for applying an electric field to the region.

The change in molecular arrangement produced by the external stimulus may be used to affect the transmission of electromagnetic radiation at any wavelength of interest through the material, for example, by changing the transmissivity of the material or by changing the helical order of the material, if it is optically active.

According to another aspect of the invention, an electro-optic liquid crystal device includes two adjacent substrates at least one of which is optically transparent, a layer of liquid crystal material contained in the space between the substrates wherein the liquid crystal material is the material as defined above, and deposited on the inner facing surfaces of the substrates, a film of conducting material to allow an electric field to be applied across the layer. The electro-optic device may for example be a display device used, for instance, in an instrument such as a time watch or clock. The device may be of known 'twisted nematic' effect or '(cholesteric-to-nematic) phase change' effect types.

Examples of methods of preparing compounds as defined will now be described. In these examples 'MP' represents melting point, 'C—N' represents crystalline to nematic liquid crystal transition temperature and 'N—I' represents nematic liquid crystal to isotropic liquid transition temperature.

Example 1

The intermediate compound 1-bromo-4-alkyl substituted bicyclo[2,2,2]octane which is used in the preparations described below may be prepared by the following route:

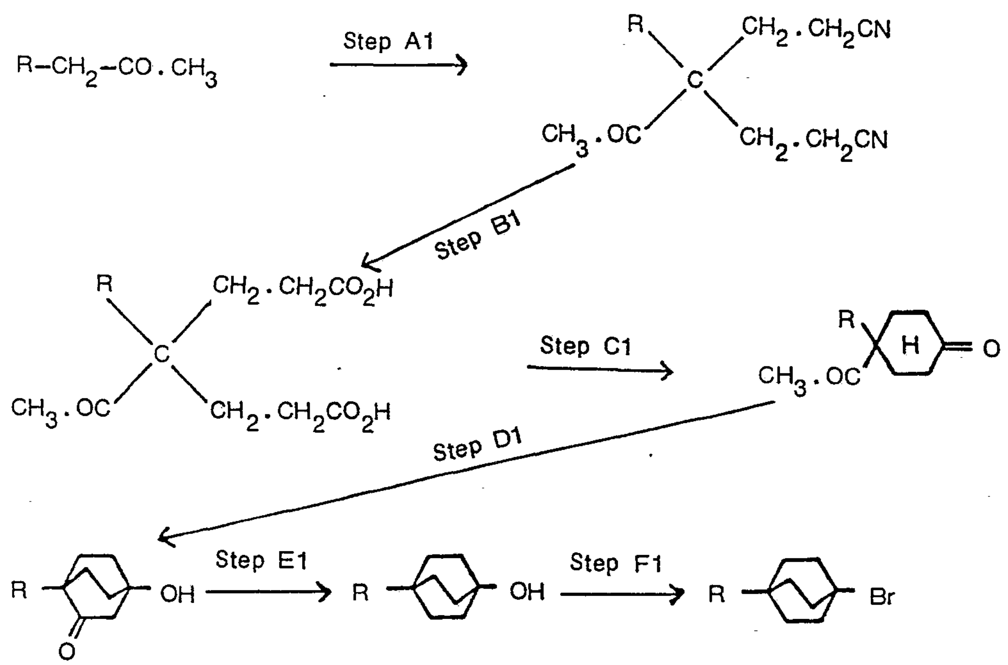


R defined as above

*Step A1:* The production of 3-acetyl-1,5-dicyano-3-substituted pentane.
*Step B1:* The production of 3-acetyl-3-substituted pentane-1,5-dicarboxylic acid.
*Step C1:* The production of 4-acetyl-4-substituted cyclohexanone.
*Step D1:* The production of 4-hydroxy-1-substituted bicyclo[2.2.2]octan-2-one.
*Step E1:* The production of 1-hydroxy-4-substituted bicyclo[2.2.2.]octane.
*Step F1:* The production of 1-bromo-4-substituted-bicyclo[2.2.2.]octane.

All six of these steps may be carried out by methods essentially analogous to those for R = methyl and ethyl described by H D Holtz and L M Stock in the Preparation of 1-Carboxy-4-Substituted Bicyclo[2.2.2]Octanes, J. Am. Chem. Soc, *86*, 5183 (1964).

Example 2

The production of 4-alkyl substituted 1-(4′-cyano-4″-biphenylyl)bicyclo[2.2.2]octane by the following route:

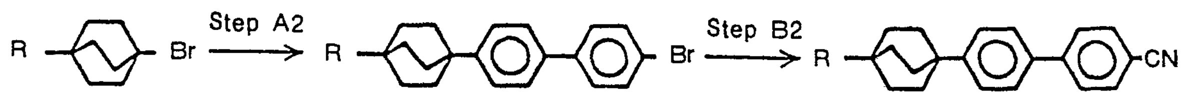


R as defined above

*Step A2:* The production of 1-(4′-bromo-4″-biphenylyl)-4-substituted bicyclo[2.2.2]octane.

A solution of 1-bromo-4-substituted bicyclo[2.2.2]octane (0.0039 mole) in dry nitrobenzene (15 $cm^3$) is added dropwise over a period of thirty minutes to a well stirred solution of crushed anhydrous aluminium chloride (0.0016 mole) and 4-bromobiphenyl (0.00116 mole) in dry nitrobenzene (25 $cm^3$) maintained at 80°C under anhydrous conditions. The mixture is kept at this temperature for five hours. It is then left stirring at room temperature, e.g. overnight. It is then added to a small amount of a hydrochloric acid/ice/water mixture and stirred for about twenty minutes. The organic layer is separated off and steam distilled to remove the solvent and 4-bromobiphenyl. The residue is dissolved in toluene and the solution so formed is dried over magnesium sulphate. It is then filtered and evaporated to dryness under vacuum. The solid residue is crystallised from ethanol and toluene. When R = pentyl, the product of this step has C—N = 192.0°C and N—I = 231.2°C.

*Step B2*: The production of 1-(4′-cyano-4″-biphenyl)-4-substituted bicyclo[2.2.2]octane.

A solution of 1-(4′-bromo-4″-biphenylyl)-4-substituted bicyclo[2.2.2]octane (0.0019) mole in dry 1-methyl-2-pyrrolidinone (5 $cm^3$) is heated at 180°C with anhydrous copper (I) cyanide (.0039 mole) for two hours under anhydrous conditions. The mixture is allowed to cool and then added to a solution of anhydrous iron (III) chloride (0.35 g) in water (5 $cm^3$) with concentrated hydrochloric acid (0.5 $cm^3$). This is stirred for twenty minutes at 50°C and then water is added (50 $cm^3$). This mixture is shaken several times with ether and then the combined organic layers are dried over magnesium sulphate. The solution is filtered and evaporated to dryness under vacuum. The product is purified by column chromatography using silica-gel with a chloroform/hexane mixture as eluent. It is finally crystallised from 2-methoxy-ethanol.

An example of such a product is:

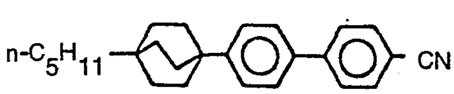

C–N = 159.7°C

N–I = 269.3°C

See also Table 2 below

Example 3

The production of 1-[4′-cyanophenyl]-4-alkyl substituted bicyclo[2.2.2]octane by the following route:

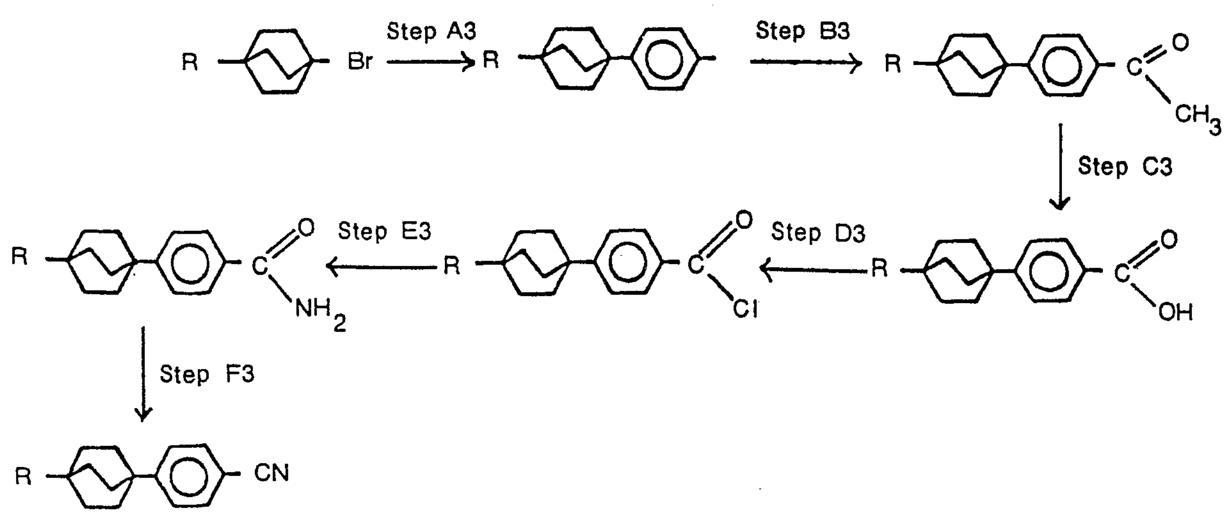


R as defined above

*Step A3*: The production of 1-phenyl-4-substituted bicyclo[2.2.2]octane may be carried out by a reaction which is analogous to that described by S Sotheeswara, ph D Thesis, Hull University Library 1967. When R = pentyl the product of this step has M.P. = 37°C.

*Step B3:* The production of 4'-(4-substituted 1-bicyclo[2.2.2]octyl)acetophenone.

A solution of acetyl chloride (0.0077) mole in dry nitrobenzene (3 $cm^3$) is added dropwise to a well stirred solution of 4-substituted 1-phenylbicyclo[2.2.2]octane (0.007 mole) and crushed anhydrous aluminium chloride (0.0082 mole) in dry nitrobenzene (7 $cm^3$) at 0°C. This solution is left stirring, e.g. overnight, at room temperature. It is then added to a small amount of a hydrochloric acid/ice/water mixture and stirred for 30 minutes. The organic layer is separated off and steam distilled. The solid residue is taken up in chloroform and the resultant solution dried over magnesium sulphate. It is then filtered and evaporated to dryness under vacuum to give a solid. This is crystallised from a methanol/ethanol mixture. When R = pentyl has the product of this step has M.P. = 81°C.

*Step C3:* The production of 4'-(4-substituted 1-bicyclo[2.2.2]octyl)benzoic acid.

A solution of sodium hypobromite, prepared by dissolving bromine (0.0168 mole) in a solution of sodium hydroxide (0.0570 mole) in water (15 $cm^3$) at 0°C, is added to a well stirred solution of 4'-(4-substituted 1-bicyclo[2.2.2]octyl)acetophenone, prepared in step B3, in dioxan (15 $cm^3$). Throughout the addition and for thirty minutes afterwards the temperature is maintained at 35—40°C. The excess of sodium hypobromite is destroyed by adding a solution of sodium metabisulphite in water. More water is added and bromoform distilled from the reaction mixture. On cooling, the solution is acidified with concentrated hydrochloric acid and the precipitated product filtered off and washed with water.

The product was crystallised from a methanol/ethanol mixture. When R = pentyl the product of this step has C—N = 226.7°C; N—I = 288.7°C.

*Step D3:* The production of 4'-(4-substituted 1-bicyclo[2.2.2]octyl)benzoyl chloride.

A solution of 4'-(4-substituted-1-bicyclo[2.2.2]octyl) benzoic acid prepared in Step C3 (0.0017 mole) in dry toluene (10 $cm^3$) is heated under reflux with thionyl chloride (1 $cm^3$) for one hour under anhydrous conditions and then allowed to cool. The mixture is evaporated to dryness and then more dry toluene (10 $cm^3$) is added. The resultant solution is again evaporated to dryness under vacuum. The acid chloride residue is used in the step E3 without further purification.

*Step E3*: The production of 4'-(4-substituted 1-bicyclo[2.2.2]octyl)benzamide.

A solution of the acid chloride prepared in step D3, in dry benzene (10 $cm^3$) is added dropwise to a well stirred ammonia solution (10 $cm^3$ d = 0.88). The resultant precipitate is collected (with some difficulty) and washed with water. It is crystallised from a water/ethanol mixture at 0°C. When R = pentyl the product of this step has M.P. = 247.3°C.

*Step F3:* The production of 1-(4'-cyanophenyl)-4- substituted bicyclo[2.2.2]octane.

A solution of 4'-(4-substituted 1-bicyclo[2.2.2]octyl)benzamide (0.0010 mole) in chloroform (18 $cm^3$) is stirred for one hour with a solution of sodium hydroxide (0.0750 mole) in water (3 $cm^3$) and 0.02 g of benzyltriethylammonium hydroxide. The mixture is gently heated for fifteen minutes in a warm water bath and then allowed to cool. Ether (40 $cm^3$) and water (40 $cm^3$) are added. The organic layer is separated off, washed with water and then dried over magnesium sulphate. The solution is filtered and evaporated to dryness under vacuum. The solid residue is purified by column chromatography using silica gel with chloroform as eluent. It is then crystallised from light petroleum (b.p. 40—60).

An example of the product of this step is n-$C_5H_{11}$

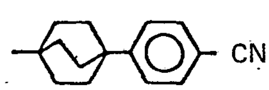

which has C—N = 62°C, N—I = 100°C (See also Table 1. below).

The following tables give examples of the liquid crystalline temperature ranges of compounds and mixtures embodying the invention. In the tables R represents a normal alkyl group having the stated composition, C—N represents solid crystal to nematic liquid crystal transition temperature (°C), C—I represents solids crystal to isotropic liquid transition temperature (°C) and N—1 represents a nematic to isotropic liquid transition temperature (°C).

TABLE 1 — Compounds having the formula. R 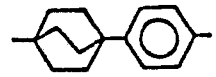 CN

| R | C—N or I | N—I |
|---|---|---|
| $CH_3$ | 93 (C—I) | (—35)* |
| $C_2H_5$ | 62.5 (C—I) | (17)* |
| $n\text{-}C_3H_7$ | 66.5 (C—N) | 88 |
| $n\text{-}C_4H_9$ | 75.5 (C—N) | 85 |
| $n\text{-}C_5H_{11}$ | 62 (C—N) | 100 |
| $n\text{-}C_6H_{13}$ | 72 (C—N) | 86 |
| $n\text{-}C_7H_{15}$ | 61 (C—N) | 95 |

( )* represents a monotropic 'virtual' transition temperature obtained by studying suitable binary mixtures of each compound with a standard material.

Further examples are $n\text{-}C_8H_{17}$ 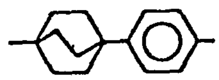 CN, $n\text{-}C_9H_{19}$ 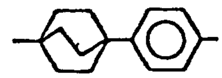 CN, $n\text{-}C_{10}H_{21}$ 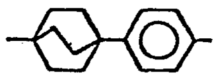 CN. Optically active analogues, e.g. $CH_3CH_2CH(CH_3)CH_2$ 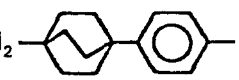 CN may be prepared by an analogous route.

TABLE 2 — Compounds having the formula R 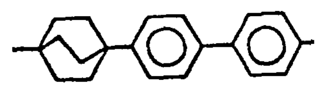 CN

| R | C—N or I | N—I |
|---|---|---|
| $CH_3$ | 198 (C—I) | (170)* |
| $C_2H_5$ | 188 (C—N) | 221 |
| $n\text{-}C_3H_7$ | 180.5 (C—N) | 276 |
| $n\text{-}C_4H_9$ | 173 (C—N) | 271.5 |
| $n\text{-}C_5H_{11}$ | 159 (C—N) | 269 |
| $n\text{-}C_6H_{13}$ | 133 (C—N) | 259 |
| $n\text{-}C_7H_{15}$ | 141 (C—N) | 250.5 |

( )* represents a monotropic 'virtual' transition temperature obtained by studying suitable binary mixtures of each compound with a standard material.

Further examples are $n\text{-}C_8H_{17}$ 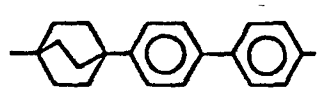 CN, $n\text{-}C_9H_{19}$ 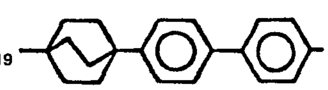 CN, and $n\text{-}C_{10}H_{21}$ 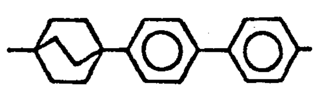 CN. Optically active analogues e.g. $CH_3CH_2CH(CH_3)CH_2$ 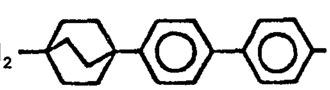 CN may be prepared by an analogous route.

Although depatures from ideality are known, examples of eutectic or nearly eutectic mixtures embodying the invention are quoted (Tables 3—6) as exemplifying the application of the above compounds in Tables 1 and 2.

TABLE 3 – The following mixture :

| Mixture Constituent | Mole % | C–N |
|---|---|---|
| 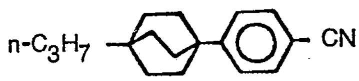 | 51.0) | 33.3 |
| 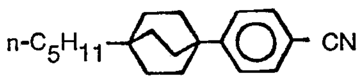 | 49.0) | |

TABLE 4 – The following mixture :

| Mixture Constituent | Mole % | C–N |
|---|---|---|
| 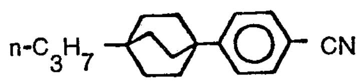 | 38.0) | 20.8 |
| 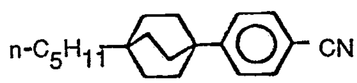 | 34.9) | |
| 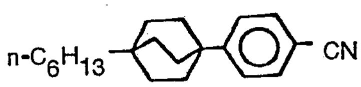 | 27.1) | |

TABLE 5 – The following mixture :

| Mixture Constituent | Mole % | C–N |
|---|---|---|
| 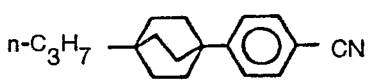 | 37.9) | 20.6 |
| 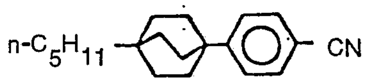 | 34.4) | |
| 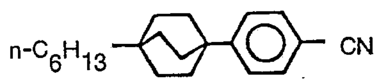 | 27.0) | |
| 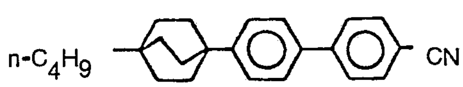 | 0.7) | |

TABLE 6 — The following mixture :

| Mixture Constituent | Mole % | C–N |
|---|---|---|
| 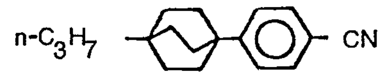 | 19.9) | −3.5 |
| 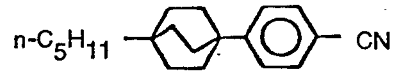 | 16.2) | |
| 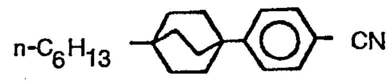 | 12.3) | |
| 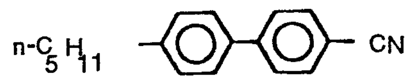 | 51.6) | |

In addition the bicyclo-octane compounds embodying the invention may be mixed with any of the compounds in one or more of the other classes listed above.

A liquid crystal device embodying the invention will now be described by way of example only with reference to the accompanying drawings of which:

Figure 1 is an exploded view of a twisted nematic display;

Figure 2 is a front view of a watch having a twisted nematic liquid crystal display constructed as shown in Figure 1; and

Figure 3 is a sectional view of the display part of the watch in Figure 2 but drawn to an enlarged scale.

As shown in Figure 1 a 'twisted nematic' type display includes a polariser 1 arranged with its polarisation axis 2 vertical. A liquid crystal cell 3 comprises two glass slides 4, 5 containing a layer of liquid crystal 6, consisting basically of a nematic material which may contain a small proportion (e.g. 1%) of a cholesteric compound. Electrodes 7, 8 e.g. of tin oxide, are arranged on the inner faces of the slides 4, 5. Behind the cell 3 is an analyser or second polariser 11 arranged with its polarisation axis 12 horizontal. A brushed aluminium reflector 27 is behind the analyser 11.

Prior to assembling the cell the slides 4, 5 are coated on their inner faces with silicon monoxide or magnesium fluoride. This coating is formed by evaporating a stream of e.g. silicon monoxide onto the slide at an angle of about 5° to the surface as for example described in UK Patent Specification No 1,454,296. On assembly the slides are arranged with the evaporation direction on the two slides 4, 5 at 90° to one another. With such a coating liquid crystal molecules at the coated surface lie in a single direction (parallel to the evaporation direction) and at an angle of about 25° to 35° typically about 30° to the slide surface. As a result the liquid crystal molecules progressively twist from one slide 4 to the other slide 5. Thus the plane of plane polarised light is rotated through 90° on passing through the cell 3 providing the direction 13 of the molecules at the surface of the slide 4, 5 is parallel (or perpendicular) the polarisers axis 2, 12 respectively. Thus when zero voltage is applied to the cell light passes through the analyser 1, cell 3 and analyser 11, and is then reflected back to an observer 15.

When a suitable voltage is applied between the electrodes 7, 8 the molecules between the electrodes are caused to align parallel to the applied field and light transmitted by the polariser is transmitted without rotation to the analyser 11. Since the polariser and analyser 11 are crossed no light is transmitted to the reflector and no light is reflected back to the observer at those parts of the cell 3 between electrodes 7, 8 which therefore appear dark on a lighter background. Thus by shaping each electrode 7, 8 into say seven discrete parts separately connectable to a voltage source the numbers 0 to 9 may be displayed.

In the watch display of Figures 2, 3 the electrodes 7, 8 are arranged to provide four seven bar numeric characters plus a period dot which may be arranged to pulse once per second.

The voltage required to align the molecules parallel to the applied field, is the 'ON' state, is dependent on layer 6 thickness, materials used, temperature and surface treatment of the slides 4, 5. A typical thicknesss of layer is 12 μm. The material used must be stable over the temperatures of use i.e. ambient temperatures which can be assumed constant for comparison purposes. A surface treatment i.e. evaporation of silicon monoxide at 5° giving large tilt e.g. 30° of molecules reduces the voltage necessary to turn the cell 'ON' at the expense of a degraded contrast or speed.

In typical watch displays using twisted nematic liquid crystal displays a 1.5 volt battery is used to drive the oscillator and logic circuits of the watch and an up converter is used to raise the voltage to 3.0 volts for operating the display.

Material embodying the invention suitable for use as the liquid crystal 6 is:

| | | |
|---|---|---|
| 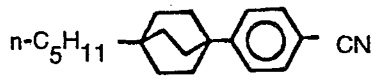 | | 10% by weight |
| 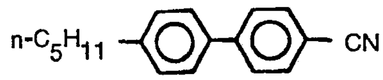 | (known) | 89% by weight |
| 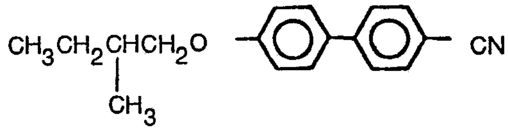 | (known) | 1% by weight |

Liquid crystal compounds and mixtures embodying the invention may also be used in known phase effect devices in which an optically active material is normally mixed with an nematic material to give a long helical Pitch cholesteric material which may be switched to a kematic state by the action of an electric field applied in a way similar to that described above in relation to the twisted nematic device. An example of a phase change effect device is described in UK Patent No 1,433,130. The compounds or mixtures embodying the invention may comprise either the nematic material (if R is n-alkyl) or the optically material (if R is a chiral alkyl group) or both to form the long pitch cholesteric material. The material may be dyed with any known suitable pleochroic dye to give enhanced contrast. Compounds and mixtures embodying the invention are particularly suitable in phase change devices in view of their low birefringence.

The following mixtures are further examples demonstrating the usefulness of the compounds and mixtures embodying the invention in twisted nematic and cholesteric-to-nematic phase change effect devices.

| Mixture 1 | wt % | N–I |
|---|---|---|
| 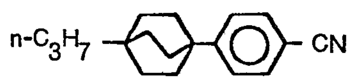 | 30 | |
| 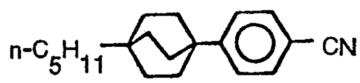 | 40 | 92.7 °C |
| 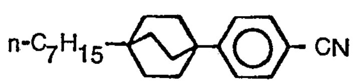 | 30 | |

*Physical Properties of Mixture 1*

Birefringence ($\Delta n$) at 20°C = 0.14

Dielectric Anisotropy ($\Delta\varepsilon$) at 20°C = 13.3

*Twisted Nematic Properties of Mixture 1*

(i) Measurements made in a 7 $\mu$m, rubbed polymer aligned cell at 20°C.

V90 (45°) = 1.35 V rms 32 Hz

V50 (10°) = 1.89 V rms 32 Hz

V10 (10°) = 2.66 V rms 32 Hz

*Key:* Vx (y) x = % Transmission, y = angle of incidence.

$$\frac{dv}{dt} (10°—80°C) = 13.4 \text{ mv/°C}$$

(ii) Response Times

$T_{Rise}$ 347 msecs }
$T_{Decay}$ 101 msecs } 3 V rms at 20°C

| Mixture 2 | wt % | N–I |
|---|---|---|
| 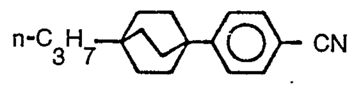 | 15 | transition |
| 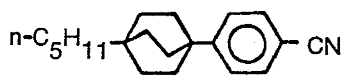 | 20 | 66°C |
| 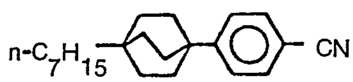 | 15 | |
| Merck ZLI 1052 (benzoate ester mixture) | 50 | |

*Physical Properties of Mixture 2*

Birefringence ($\Delta_n$) at 20°C = 0,14

*Twisted Nematic Properties of Mixture 2*

(i) Measurements made in a 7 $\mu$m, rubbed polymer aligned cell at 20°C.

V90 (45°) = 1.30 V rms at 32 Hz

V50 (10°) = 1.73 V rms at 32 Hz

V10 (10°) = 2.28 V rms at 32 Hz

V90, V50 and V10 are the voltages for 90, 50 and 10% transmission respectively. The figures in brackets are the angles between the axis of measurement and the normal to the twisted nematic cell.

The rate of change of voltage with temperature $\left(\frac{DV}{dt}\right)$ 0—40°C = 10.3 mv/°C.

(ii) Response Times

$T_{Rise}$ 172 ms }
$T_{Decay}$ 92 ms } 3 V rms at 20°C

Mixture 3

(Long Pitch Cholesteric)

| | wt. % | Ch—I |
|---|---|---|
| Mixture 1 | 96.4 | transition) |
| + | | |
| 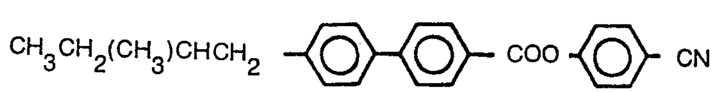 | 3.6 | 97°C |

CH = cholesteric, I = isotropic liquid, Pitch length = 5$\mu$m.

*Phase Change Properties*

(i) Von = 7.8 V rms 12 $\mu$m cell homeotropic boundary conditions, measured at 23°C.

(ii) Response Times

$T_{Rise}$ 450 ms, $T_{Decay}$ 400 ms — at 10 V, 23°C

**Claims**

1. A liquid crystal compound having a molecular structure

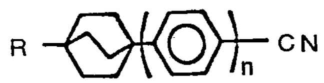

where R is an alkyl group and

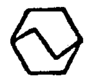

is a bicyclo(2.2.2)octane ring, n being an integer greater than zero.

2. A liquid crystal compound as claimed in claim 1 and wherein R is an n-alkyl group having not more than 18 carbon atoms.

3. A liquid crystal compound as claimed in claim 1 and wherein R is a branched alkyl group having not more than 18 carbon atoms and containing a chiral centre.

4. A liquid crystal compound as claimed in claim 1 and wherein the integer n is one, the compound being a 1-[4'cyanophenyl]-4-alkyl substituted bicyclo[2.2.2]octane.

5. A liquid crystal compound as claimed in claim 1 and wherein the integer n is two, the compound being a 1-[4'-cyano-4''-biphenylyl]-4-alkyl substituted bicyclo[2.2.2]octane.

6. A liquid crystal compound as claimed in claim 4 and wherein R is an n-alkyl group having from 1 to 10 carbon atoms.

7. A liquid crystal compound as claimed in claim 4 and wherein R is an alkyl group having from 3 to 10 carbon atoms and containing a chiral centre.

8. A liquid crystal compound as claimed in claim 5 and wherein R is an n-alkyl group having from 1 to 10 carbon atoms.

9. A liquid crystal compound as claimed in claim 5 and wherein R is an alkyl group having from three to ten carbon atoms and containing a chiral centre.

10. A liquid crystal compound as claimed in claim 6 and wherein the compound is

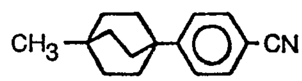

11. A liquid crystal compound as claimed in claim 6 and wherein the compound is

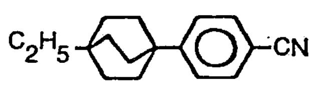

12. A liquid crystal compound as claimed in claim 6 and wherein the compound is

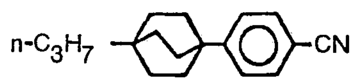

13. A liquid crystal compound as claimed in claim 6 and wherein the compound is

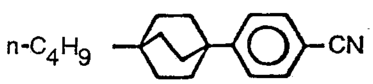

14. A liquid crystal compound as claimed in claim 6 and wherein the compound is

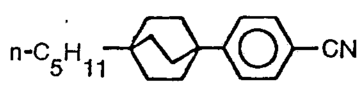

15. A liquid crystal compound as claimed in claim 6 and wherein the compound is

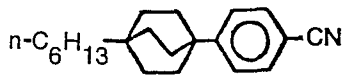

16. A liquid crystal compound as claimed in claim 6 and wherein the compound is

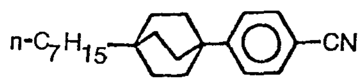

17. A liquid crystal compound as claimed in claim 8 and wherein the compound is

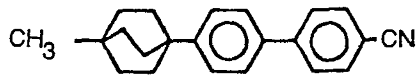

18. A liquid crystal compound as claimed in claim 8 and wherein the compound is

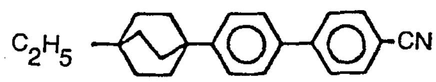

19. A liquid crystal compound as claimed in claim 8 and wherein the compound is

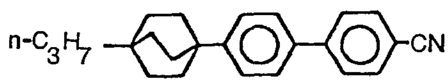

20. A liquid crystal compound as claimed in claim 8 and wherein the compound is

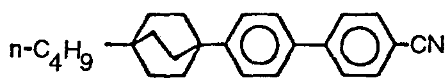

21. A liquid crystal compound as claimed in claim 8 and wherein the compound is

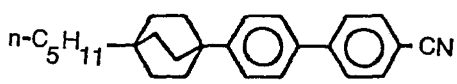

22. A liquid crystal compound as claimed in claim 8 and wherein the compound is

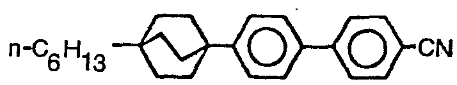

23. A liquid crystal compound as claimed in claim 8 and wherein the compound is

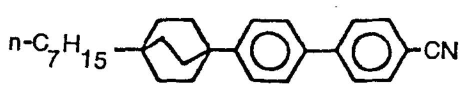

24. A liquid crystal material comprising a mixture of compounds at least one of which is a compound as claimed in claim 1.

25. A liquid crystal material comprising a mixture of compounds at least two of which are compounds as claimed in claim 1.

26. A liquid crystal material as claimed in claim 25 and wherein the mixture contains at least one compound of the form

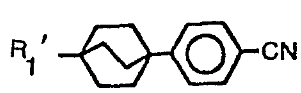

and at least one compound of the form

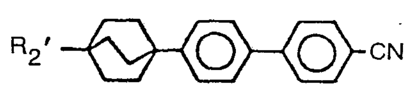

wherein $R'_1$ and $R'_2$ are n-alkyl groups.

27. A liquid crystal material as claimed in claim 24 and wherein the mixture contains at least one compound of the form

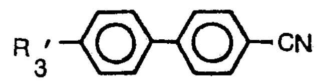

where $R'_3$ is an alkyl or alkoxy group.

28. An electro-optic liquid crystal device including two adjacent substrates at least one of which is optically transparent, a layer of liquid crystal material contained in the space between the substrates and, deposited on the inner facing surfaces of the substrates, a film of conductng material to allow an electric field to be applied across the layer and characterised in that the liquid crystal material includes a compound as claimed in claim 1.

29. A device as claimed in claim 28 and wherein the liquid crystal device is a twisted nematic effect device.

30. A device as claimed in claim 28 and wherein the liquid crystal device is a cholesteric-to-nematic phase change effect device.

**Patentansprüche**

1. Eine Flüssigkristallverbindung mit der Molekülstruktur

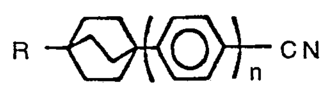

wobei R eine Alkyl gruppe und

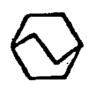

ein bizyklischer Oktanring (2.2.2) ist; n ist eine Ganzzahl größer als Null.

2. Eine Flüssigkristallverbindung gemäß Anspruch 1, wobei R eine n-Alkylgruppe mit nicht mehr als 18 Kohlenstoffatomen ist.

3. Eine Flüssigkristallverbindung gemäß Anspruch 1, wobei R eine verzweigte Alkylgruppe mit nicht mehr als 18 Kohlenstoffatomen ist, die ein chirales Zentrum enthält.

4. Eine Flüssigkristallverbindung gemäß Anspruch 1, wobei die Ganzzahl n 1 beträgt, und die Verbindung ist ein 1-(4′-Zyanphenyl)-4-Alkylsubstituiertes bizyklisches Oktan (2.2.2).

5. Eine Flüssigkristallverbindung gemäß Anspruch 1, wobei die Ganzzahl n 2 beträgt und die Verbindung ein 1-(4′-Zyan-4″-Biphenylyl)-4-Alkyl-substituiertes bizyklisches Oktan (2.2.2) ist.

6. Eine Flüssigkristallverbindung gemäß Anspruch 4, wobei R eine n-alkylgruppe mit 1 bis 10 Kohlenstoffatomen ist.

7. Eine Flüssigkristallverbindung gemäß Anspruch 4, wobei R eine Alkylgruppe mit 3 bis 10 Kohlenstoffatomen ist, die ein chirales Zentrum enthält.

8. Eine Flüssigkristallverbindung gemäß Anspruch 5, wobei R eine n-Alkylgruppe mit 1 bis 10 Kohlenstoffatomen ist.

9. Eine Flüssigkristallverbindung gemäß Anspruch 5, wobei R eine Alkylgruppe mit 3 bis 10 Kohlenstoffatomen ist, die ein chirales Zentrum enthält.

10. Eine Flüssigkristallverbindung gemäß Anspruch 6 mit nachstehendem Molekülbau:

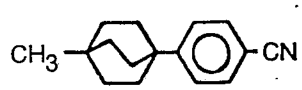

11. Eine Flüssigkristallverbindung gemäß Anspruch 6 mit nachstehendem Molekülbau:

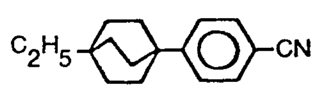

12. Eine Flüssigkristallverbindung gemäß Anspruch 6 mit nachstehendem Molekülbau:

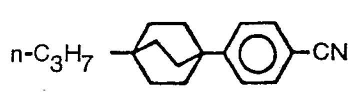

13. Eine Flüssigkristallverbindung gemäß Anspruch 6 mit nachstehendem Molekülbau:

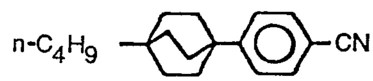

14. Eine Flüssigkristallverbindung gemäß Anspruch 6 mit nachstehendem Molekülbau:

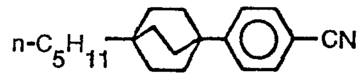

15. Eine Flüssigkristallverbindung gemäß Anspruch 6 mit nachstehendem Molekülbau:

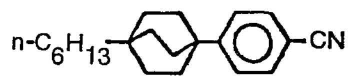

16. Eine Flüssigkristallverbindung gemäß Anspruch 6 mit nachstehendem Molekülbau:

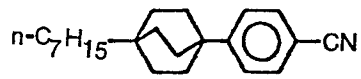

17. Eine Flüssigkristallverbindung gemäß Anspruch 8 mit nachstehendem Molekülbau:

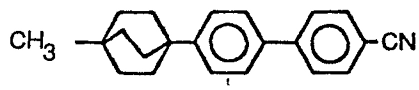

18. Eine Flüssigkristallverbindung gemäß Anspruch 8 mit nachstehendem Molekülbau:

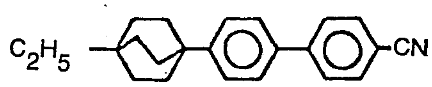

19. Eine Flüssigkristallverbindung gemäß Anspruch 8 mit nachstehendem Molekülbau:

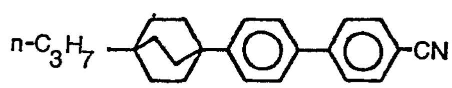

20. Eine Flüssigkristallverbindung gemäß Anspruch 8 mit nachstehendem Molekülbau:

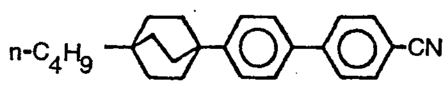

21. Eine Flüssigkristallverbindung gemäß Anspruch 8 mit nachstehendem Molekülbau:

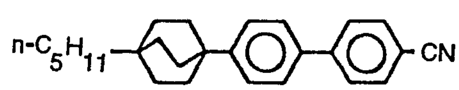

22. Eine Flüssigkristallverbindung gemäß Anspruch 8 mit nachstehendem Molekülbau:

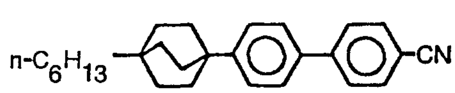

23. Eine Flüssigkristallverbindung gemäß Anspruch 8 mit nachstehendem Molekülbau:

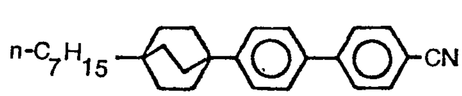

24. Ein Flüssigkristallmaterial, bestehend aus einer Mischung von Verbindungen, die mindestens eine Verbindung enthält, die Anspruch 1 entspricht.

25. Ein Flüssigkristallmaterial, bestehend aus einer Mischung von Verbindungen, die mindestens zwei Verbindungen enthält, die Anspruch 1 entsprechen.

26. Ein Flüssigkristallmaterial gemäß Anspruch 25, wobei die Mischung mindestens eine Verbindung der Formel

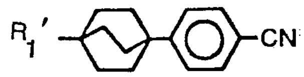

und mindestens eine Verbindung der Formel

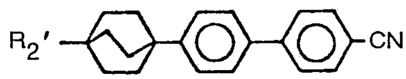

enthält, wobei $R'_1$ und $R'_2$ n-Alkylgruppen sind.

27. Ein Flüssigkristallmaterial gemäß Anspruch 24, wobei die Mischung mindestens eine Verbindung der Formel

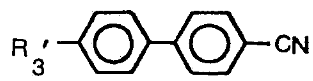

enthält, und wo $R'_3$ eine Alkyl- oder Alkoxygruppe ist.

28. Ein elektro-optisches Flüssigkristallgerät, bestehend aus zwei benachbarten Substratschichten, von denen mindestens eine optisch transparent ist, einer im Spalt zwischen den Substratschichten enthaltenen Lage von Flüssigkristallmaterial und einem auf der inneren Oberfläche der Substratschichten abgelagerten Film aus leitendem Werkstoff, der es ermöglicht, ein elektrisches Feld über diesen Schichten anzulegen, wobei es für dieses Gerät charakteristisch ist, daß das Flüssigkristallmaterial eine Verbindung gemäß Anspruch 1 enthält.

29. Ein Gerät gemäß Anspruch 28, wobei es sich bei dem Flüssigkristallgerät um ein Gerät mit nematischer Drehwirkung handelt.

30. Ein Gerät gemäß Anspruch 28, wobei es sich bei dem Flüssigkristallgerät um ein Gerät mit cholesterisch-nematischer Phasenwandlungswirkung handelt.

**Revendications**

1. Un composé de cristaux liquides ayant une structure moléculaire

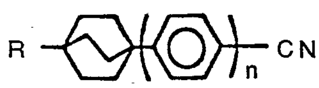

où R est un groupe alcoyle et

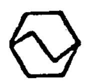

est un noyau bicyclo (2.2.2) octane, n étant (un nombre entier) plus grand que zéro.

2. Un composé de cristaux liquides tel que revendiqué par la revendication 1 et dans lequel R est un groupe n-alcoyle ne possédant pas plus de 18 atomes de carbone.

3. Un composé de cristaux liquides tel que revendiqué par la revendication 1 et dans lequel R est un groupe alcoyle ramifié ne possédant pas plus de 18 atomes de carbone et renfermant un centre chiral.

4. Un composé de cristaux liquides tel que revendiqué par la revendication 1 et dans lequel (le nombre entier) n est un, le composé étant un bicyclo[2.2.2]octane substitué en (position) 1-par (le radical)[4'cyanophényle]-4-alcoyle.

5. Un composé de cristaux liquides tel que revendiqué par la revendication 1 et dans lequel (le nombre entier) n est deux, le composé étant un bicyclo[2.2.2]octane substitué en 1 par [4'-cyano-4"-bi-phényle]-4-alcoyle.

6. Un composé de cristaux liquides tel que revendiqué par la revendication 4 et dans lequel R est un groupe n-alcoyle possédant de 1 à 10 atomes de carbone.

7. Un composé de cristaux liquides tel que revendiqué par la revendication 4 et dans lequel R est un groupe alcoyle possédant de trois à 10 atomes de carbone et renfermant un centre chiral.

8. Un composé de cristaux liquides tel que revendiqué par la revendication 5 et dans lequel R est un groupe n-alcoyle possédant de 1 à 10 atomes de carbone.

9. Un composé de cristaux liquides tel que revendiqué par la revendication 5 et dans lequel R est un groupe alcoyle possédant de trois à dix atomes de carbone et renfermant un centre chiral.

10. Un composé de cristaux liquides tel que revendiqué par la revendication 6 et dans lequel le composé est

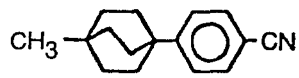

11. Un composé de cristaux liquides tel que revendiqué par la revendication 6 et dans lequel le composé est

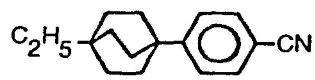

12. Un composé de cristaux liquides tel que revendiqué par la revendication 6 et dans lequel le composé est

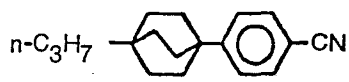

13. Un composé de cristaux liquides tel que revendiqué par la revendication 6 et dans lequel le composé est

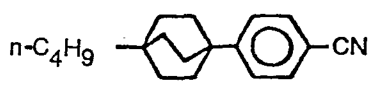

14. Un composé de cristaux liquides tel que revendiqué par la revendication 6 et dans lequel le composé est

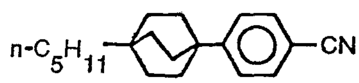

15. Un composé de cristaux liquides tel que revendiqué par la revendication 6 et dans lequel le composé est

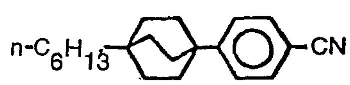

16. Un composé de cristaux liquides tel que revendiqué par la revendication 6 et dans lequel le composé est

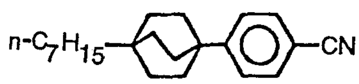

17. Un composé de cristaux liquides tel que revendiqué par la revendication 8 et dans lequel le composé est

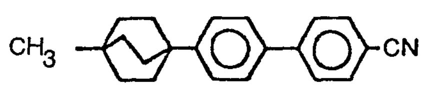

18. Un composé de cristaux liquides tel que revendiqué par la revendication 8 et dans lequel le composé est

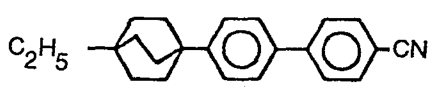

19. Un composé de cristaux liquides tel que revendiqué par la revendication 8 et dans lequel le composé est

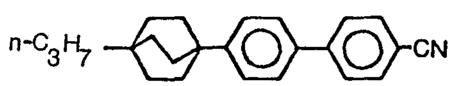

20. Un composé de cristaux liquides tel que revendiqué par la revendication 8 et dans lequel le composé est

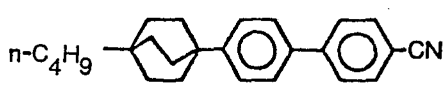

21. Un composé de cristaux liquides tel que revendiqué par la revendication 8 et dans lequel le composé est

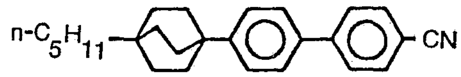

22. Un composé de cristaux liquides tel que revendiqué par la revendication 8 et dans lequel le composé est

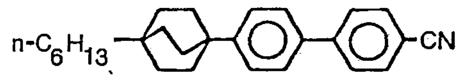

23. Un composé de cristaux liquides tel que revendiqué par la revendication 8 et dans lequel le composé est

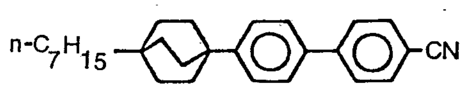

24. Un matériau de cristaux liquides renfermant un mélange de composés dont au moins un est un composé tel que revendiqué par la revendication 1.

25. Un matériau de cristaux liquides renfermant un mélange de composés dont au moins deux sont des composés tels que (celui qui est) revendiqué par la revendication 1.

26. Un matériau de cristaux liquides tel que revendiqué par la revendication 25 et dans lequel le mélange contient au moins un composé (dont la structure moléculaire) a la configuration

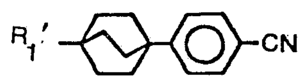

et au moins un composé (dont la structure moléculaire) a la configuration

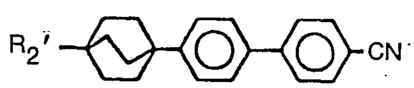

où $R'_1$ et $R'_2$ sont des groupes n-alcoyle.

27. Un matériau de cristaux liquides tel que revendiqué par la revendication 24 et dans lequel le mélange contient au moins un composé (dont la structure moléculaire) a la configuration

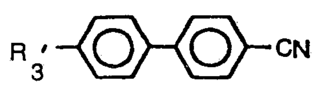

où $R'_3$ est un groupe alcoyle ou alcoxyle.

28. Un dispositif électro-optique à cristaux liquides comprenant deux substrats adjacents dont au moins un est optiquement transparent, une couche de matériau à cristaux liquides enfermé dans l'espace entre les substrats et, déposé sur l'inter-face des substrats, une pellicule de matériau conducteur afin de permettre l'application d'un champ électrique à travers la couche et caractérisé en ce que le matériau de cristaux liquides comprend un composé tel que revendiqué par la revendication 1.

29. Un dispositif tel que revendiqué par la revendication 28 et dans lequel le dispositif à cristaux liquides est un dispositif à effet nématique vrillé.

30. Un dispositif tel que revendiqué par la revendication 28 et dans lequel le dispositif à cristaux liquides est un dispositif à effet de changement de phase cholestérique-à-nématique.

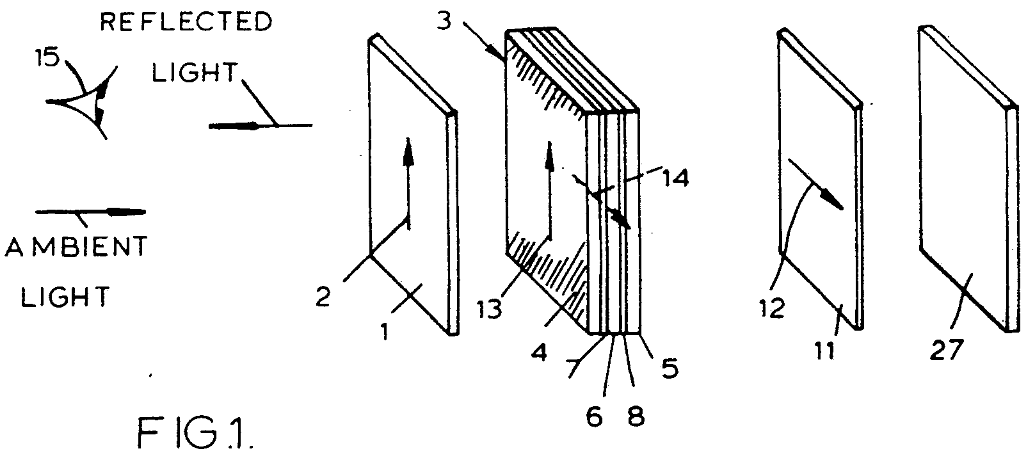


FIG.1.

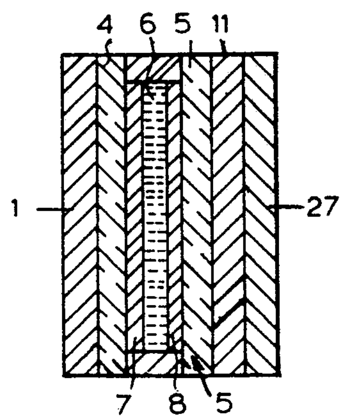


FIG.3

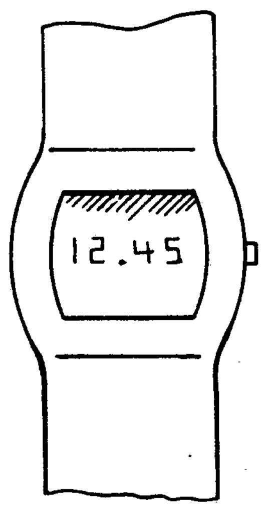


FIG.2.